# EUROPÄISCHE PATENTSCHRIFT

(11) **EP 2 629 816 B1**
(45) Veröffentlichungstag und Bekanntmachung des Hinweises auf die Patenterteilung: **17.05.2017**
(21) Anmeldenummer: 11776726.9
(22) Anmeldetag: 30.09.2011
(51) Int. Cl.: C12M 1/00, A61M 1/36, A61M 1/00

(54) **VERFAHREN UND VORRICHTUNG ZUM SEPARIEREN VON ADULTEN STAMMZELLEN AUS FETTGEWEBE**
METHOD AND APPARATUS FOR SEPARATING ADULT STEM CELLS FROM ADIPOSE TISSUE
PROCEDE ET DISPOSITIF DE SEPARATION DE CELLULES SOUCHES PROVENANT DU TISSU ADIPEUX

(30) Priorität: 27.09.2011 US 201113246275; 02.08.2011 DE 102011080218; 21.10.2010 US 405246 P; 20.10.2010 DE 102010049203
(43) Veröffentlichungstag der Anmeldung: 28.08.2013
(73) Patentinhaber: Human Med AG, 19061 Schwerin (DE)
(72) Erfinder: KENSY, Arnd, 14552 Michendorf - OT Wilhelmshorst (DE); WINKLER, Konrad-Wenzel, 19417 Warin (DE)
(74) Vertreter: Gulde & Partner
(86) Internationale Anmeldenummer: PCT/EP2011/067115
(87) Internationale Veröffentlichungsnummer: WO 2012/052275

(56) Entgegenhaltungen:
- WO-A1-2008/143570
- WO-A1-2010/045389
- WO-A1-2010/073808
- WO-A2-2007/009036
- JP-A- 2010 104 918
- BUNNELL B A ET AL: "Adipose-derived stem cells: Isolation, expansion and differentiation", METHODS : A COMPANION TO METHODS IN ENZYMOLOGY, ACADEMIC PRESS INC., NEW YORK, NY, US, Bd. 45, Nr. 2, 1. Juni 2008 (2008-06-01), Seiten 115-120, XP022796172, ISSN: 1046-2023, DOI: 10.1016/J.YMETH.2008.03.006 [gefunden am 2008-05-29]
- UEBERREITER K ET AL: "[BEAULI(TM)--a new and easy method for large-volume fat grafts].", HANDCHIRURGIE, MIKROCHIRURGIE, PLASTISCHE CHIRURGIE : ORGAN DER DEUTSCHSPRACHIGEN ARBEITSGEMEINSCHAFT FÜR HANDCHIRURGIE : ORGAN DER DEUTSCHSPRACHIGEN ARBEITSGEMEINSCHAFT FÜR MIKROCHIRURGIE DER PERIPHEREN NERVEN UND GEFÄSSE : ORGAN DER VEREINIGUNG DER, vol. 42, no. 6, December 2010 (2010-12), pages 379-385, ISSN: 1439-3980

## Beschreibung

Die Erfindung betrifft ein Verfahren und eine Vorrichtung zum Separieren von adulten Stammzellen aus Fettgewebe.

Es ist bekannt, Fettgewebe aus biologischen Strukturen beispielsweise in der Humanmedizin zu gewinnen. Fettgewebe wird bekanntermaßen durch geeignete Absaugeinrichtungen abgesaugt und in so genannten Fettgewebezellensammlern gesammelt.

Bekannt ist, dass das so gewonnene Fettgewebe neben Fettzellen auch Stammzellen umfasst. Stammzellen sind Körperzellen, die sich in verschiedenen Zelltypen oder Gewebe ausdifferenzieren können. Bei den im Fettgewebe vorhandenen Stammzellen handelt es sich um so genannte adulte Stammzellen

Zur Gewinnung derartiger adulter Stammzellen aus Fettgewebe ist bekannt, beispielsweise aus WO 2010/073808 A1, das Fettgewebe mit einem Enzym enthaltenden Fluid zu versetzen und das Gemisch anschließend in einem Behälter zu zentrifugieren. Hierdurch erfolgt ein Lösen der Stammzellen von den Fettzellen, so dass diese gesondert gesammelt werden können.

Aus WO 2010/045 389 A1 ist eine Methode und eine Vorrichtung bekannt, um muskuläres Fettgewebe - welches mit adulten Stammzellen angereichert ist - vom restlichen Fettgewebe zu trennen. Das Fettzellen-/Stammzellengemisch wird in einer Presse einem mechanischen Druck ausgesetzt, um eine Fasentrennung von Fett und den schwereren Zellen zu erreichen.

Der Erfindung liegt die Aufgabe zugrunde, ein Verfahren und eine Vorrichtung der gattungsgemäßen Art zu schaffen, mittels denen in einfacher Weise eine Separation von adulten Stammzellen aus Fettgewebe erfolgen kann.

Die Aufgabe wird durch den Gegenstand der vorliegenden Erfindung gelöst, nämlich eine Vorrichtung zum Separieren von adulten Stammzellen aus Fettgewebe. Erfindungsgemäß ist vorgesehen, dass die Vorrichtung einen Behälter zur Aufnahme von aus einer biologischen Struktur entnommenem Fettgewebe, das Fettzellen und Stammzellen umfasst, eine Zuführeinrichtung für ein unter Druck stehendes Fluid, eine Austrittsanordnung für das unter Druck stehende Fluid, wobei die Austrittsanordnung von einem Hohlkörper gebildet wird und die Austrittsanordnung innerhalb des Behälters derart angeordnet ist, dass der die Austrittsanordnung bildende Hohlkörper eine Ausgangsöffnung und eine Eingangsöffnung besitzt, in die das die Fettzellen und die Stammzellen enthaltende Fettgewebe durch den Fluidstrahl eingesaugt wird. Hierdurch wird vorteilhaft möglich, das in dem Behälter befindliche, die adulten Stammzellen und Fettzellen umfassende Fettgewebe mit einem Fluidstrahl zu beaufschlagen. Der Fluidstrahl bewirkt, bei entsprechendem Druck, eine Trennung der adulten Stammzellen von den Fettzellen. Gleichzeitig bewirkt der Fluidstrahl eine innerhalb des Behälters sich einstellende Strömungsrichtung des gesamten Gemisches, so dass immer wieder neue mit adulten Stammzellen verbundene Fettzellen in den Wirkbereich des Fluidstrahles gelangen. Hierdurch wird eine sehr effektive Separation der adulten Stammzellen von Fettzellen erreicht. Aufgrund der unterschiedlichen Dichte sinken die adulten Stammzellen innerhalb des Gemisches ab und können so separat entnommen werden.

Wie oben beschrieben umfasst die Austrittsanordnung erfindungsgemäß den Hohlkörper mit der Eingangsöffnung und der Ausgangsöffnung, wobei der Fluidstrahl durch die Austrittsanordnung hindurchtritt. Hierdurch wird vorteilhaft erreicht, dass die Austrittsanordnung gleichzeitig eine definierte Mischkammer für das Fettgewebe mit dem eingebrachten Fluid bildet.

Der Hohlkörper ist vorzugsweise als Hohlzylinder ausgebildet. Hierdurch kann einerseits durch den Fluidstrahl eine definierte Strömung in dem Fettgewebe-Fluid-Gemisch aufweisenden Behälter erzeugt werden und andererseits ein Raum geschaffen werden innerhalb dem das Abwaschen der adulten Stammzellen von den Fettzellen erfolgt. Über Form und Größe des Hohlkörpers, insbesondere des Hohlzylinders, kann auf die Wirkung des Fluidstrahles auf das Fettgewebe Einfluss genommen werden.

In weiter bevorzugter Ausgestaltung der Erfindung ist vorgesehen, dass die Austrittsanordnung Strömungsflächen aufweist. Diese Strömungsflächen können von dem Fluidstrahl angeströmt werden, so dass dieser umgelenkt wird. Hierdurch wird vorteilhaft möglich das Fettgewebe innerhalb des der Austrittsanordnung zu verwirbeln, so dass ein effektiveres Auswaschen der adulten Stammzellen von den Fettzellen möglich ist.

Ferner ist in bevorzugter Ausgestaltung der Erfindung vorgesehen, dass die Austrittsanordnung eine Düse, insbesondere eine Venturidüse ausgebildet. Hierdurch wird vorteilhaft erreicht, dass durch Einbringen des Fluidstrahles in die Austrittsanordnung durch diese gleichzeitig das die Austrittsanordnung umgebende Fettgewebe mit in die Austrittsanordnung eingesogen wird. Zusätzliche Maßnahmen zum Einbringen des Fettgewebes in die Austrittanordnung, die gleichzeitig die Behandlungskammer zum Auswaschen der Stammzellen bildet, sind nicht erforderlich. Die gesamte Vorrichtung zum Separieren der adulten Stammzellen kann somit einen einfachen und kompakten Aufbau besitzen.

Die Aufgabe wird ferner durch ein erfindungsgemäßes Verfahren zum Separieren von adulten Stammzellen mit folgenden Verfahrensschritten gelöst:
- Einbringen eines die Stammzellen und das Fettgewebe umfassenden Fettgewebeflüssigkeitsgemisches in einen Behälter einer erfindungsgemäßen Vorrichtung gemäß der obigen Beschreibung,
- Zuführen eines unter Druck zwischen 2 und 10 bar stehenden Fluidstrahles in das Fettzellen und Stammzellen enthaltende Fettgewebe,
- Trennen der Stammzellen von den Fettzellen durch eine Strahlwirkung des Fluidstrahls,
- Separieren der Stammzellen.

Die adulten Stammzellen werden von den Fettzellen mechanisch separiert und anschließend getrennt von den Fettzellen. Hierdurch wird ein Auswaschen der Stammzellen aus dem Fettgewebe möglich. Durch Beaufschlagung mit einem unter Druck stehenden Fluidstrahl erfolgt eine Trennung der adulten Stammzellen von den Fettzellen. In dem sich durch das Einbringen von Fluid einstellenden, Fettzellen, adulte Stammzellen und Fluid enthaltenden, Gemisch sinken aufgrund der größeren Dichte die adulten Stammzellen ab, während die Fettzellen aufgrund ihrer geringeren Dichte gegenüber dem eingesetzten Fluid aufschwimmen. Hierdurch erfolgt eine Trennung der Fettzellen von den adulten Stammzellen durch Sinkfraktion. Die adulten Stammzellen können somit separiert, beispielsweise durch Absaugen oder dergleichen, und gesammelt werden. Vorzugsweise können die Stammzellen anschließend aus dem Stammzellen-Fluid-Gemisch herausgefiltert werden.

Weitere bevorzugte Ausgestaltung der Erfindung ergeben sich aus den übrigen, in den Unteransprüchen genannten Merkmalen.

Die Erfindung wird nachfolgend in einem Ausführungsbeispiel anhand der zugehörigen Zeichnungen näher erläutert. Es zeigen:
- Figur 1: eine schematische Seitenansicht der erfindungsgemäßen Vorrichtung zum Separieren von adulten Stammzellen;
- Figur 2: eine schematische Draufsicht auf die Vorrichtung;
- Figur 3: eine schematische Schnittdarstellung durch eine Austrittsanordnung;
- Figur 4: mehrere Ausgestaltungsmöglichkeiten der Austrittsanordnung in schematischen Ansichten und
- Figur 5: eine schematische Ansicht von Fettzellen.

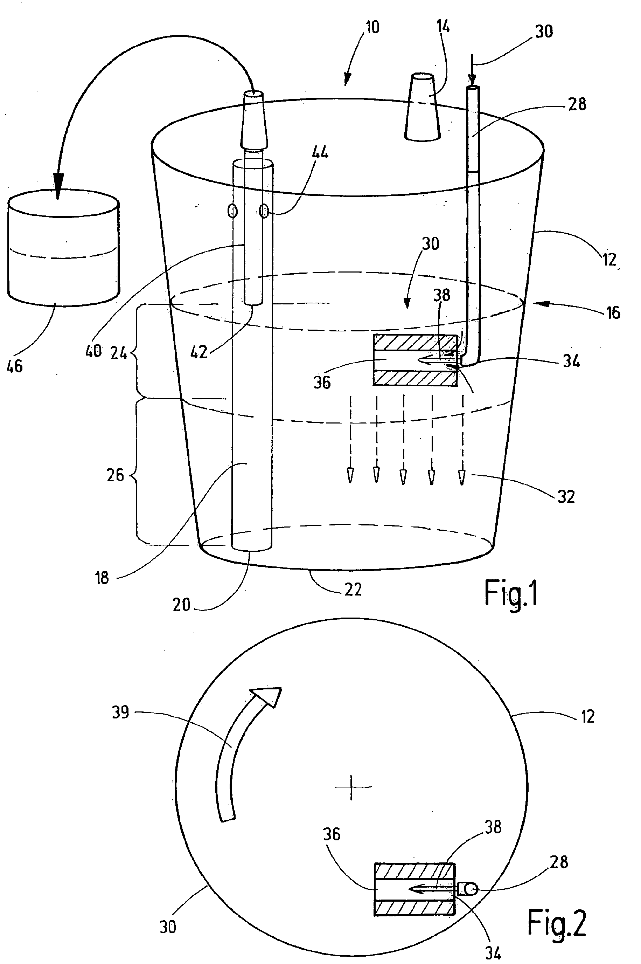

In Figur 1 ist schematisch eine Vorrichtung 10 zur Separation von adulten Stammzellen aus Fettgewebe gezeigt. Die Vorrichtung 10 umfasst einen Behälter 12, in dem über einen hier angedeuteten Anschlussstutzen 14 aus einer nicht dargestellten biologischen Struktur entnommenes Fettgewebe eingebracht ist. Das Fettgewebe wird üblicherweise durch eine so genannte Wasserstrahlchirurgie der biologischen Struktur entnommen und liegt als Fettgewebeflüssigkeitsgemisch vor. Entsprechend der eingebrachten Menge des Fettgewebeflüssigkeitsgemisches stellt sich ein Pegel, hier mit 16 bezeichnet, innerhalb des Behälters 12 ein. Innerhalb des Behälters 12 ist ein mit einer nicht dargestellten Unterdruckquelle verbundenes Rohr 18 eingesetzt, dessen Mündung 20 kurz oberhalb eines Bodens 22 des Behälters 12 liegt.

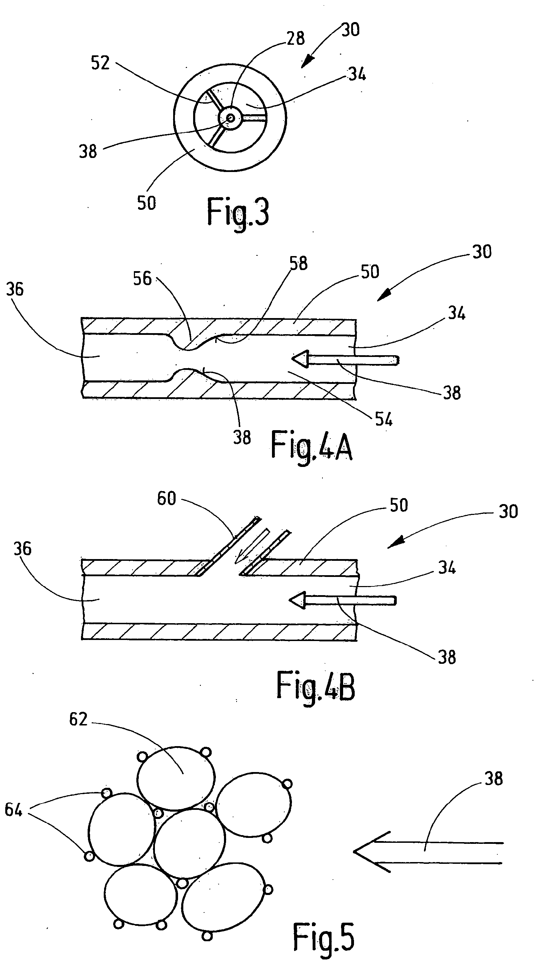

Aufgrund der Dichte der Fettzellen bildet sich eine Schicht 24 von Fettzellen des Fettgewebes und eine Schicht 26 der Flüssigkeit. Die Fettzellen in der Schicht 24 sind mit adulten Stammzellen vermischt, so wie diese aus der biologischen Struktur gewonnen wurden. Üblicherweise docken die räumlich sehr viel kleineren adulten Stammzellen an den größeren Fettzellen an deren Oberfläche an und haften an diesen (Figur 5).

Die Vorrichtung 10 umfasst ferner eine Zuführeinrichtung 28 für ein unter Druck stehendes Fluid, beispielsweise Wasser. Über eine nicht dargestellte Schalteinrichtung kann der Fluidzufluss zum Behälter 12 gestartet, unterbrochen, gedrosselt oder abgeschaltet werden. Die Zuführeinrichtung umfasst eine hier angedeutete Austrittsanordnung 30, mittels der der Fluidstrahl in die Schicht 24, die Fettzellen umfasst, eingebracht wird. Die Austrittsanordnung ist über nicht dargestellte Verbindungsmittel mit der Zuführeinrichtung 28 verbunden, so dass eine gemeinsame Einheit gegeben ist.

Der Fluidstrahl besitzt einen solchen Druck, beispielsweise zwischen 2 und 10 bar, insbesondere zwischen 3 und 4 bar, dass die adulten Stammzellen von den Fettzellen getrennt werden. Diese werden quasi durch die Strahlwirkung des Fluidstrahles abgewaschen. Die so vereinzelten Stammzellen sinken durch ihre größere Dichte - wie hier mit den Pfeilen 32 angedeutet - innerhalb der Schicht 26 in Richtung des Bodens 22 ab.

Die Austrittsanordnung 30 ist als Hohlkörper, beispielsweise als Hohlzylinder ausgebildet und besitzt eine Eingangsöffnung 34 und eine Ausgangsöffnung 36. Durch Einbringen des Fluidstromes, hier mit 38 gekennzeichnet, werden die Fettzellen über die Eingangsöffnung 34 in die Austrittsanordnung 30 eingesaugt und treffen dort auf den Fluidstrahl 38. Die Fettzellen werden somit mitgerissen und es erfolgt die bereits erwähnte Abwaschung der adulten Stammzellen von den Fettzellen. Der Fluidstrahl 38 erzeugt gleichzeitig eine gerichtete Strömung innerhalb der gesamten Schicht 24 der Fettzellen.

Wie die schematische Draufsicht in Figur 2 verdeutlicht, befindet sich die Austrittsanordnung 30 in einem radial äußeren Bereich des Behälters 12. Hierdurch wird erreicht, dass sich innerhalb der Schicht 24 eine Grundströmung einstellt, die hier mit dem Pfeil 39 angedeutet ist. Somit werden die Fettzellen innerhalb der Schicht 24 vermischt bzw. bewegt und gelangen nach einer Zeit in den Bereich der Austrittsanordnung 30. Hierdurch kann ein möglichst vollständiges Auswaschen der adulten Stammzellen von den Fettzellen erfolgen.

Die adulten Stammzellen sinken, wie bereits erläutert, auf den Boden 22 des Behälters 12 ab und werden dort über das Rohr 18 mit der Flüssigkeit abgesaugt. Hierzu ist innerhalb des Rohres 18 ein Saugstutzen 40 angeordnet, dessen Saugmündung 42 die Höhe des Pegels 16 bestimmt. Über im Rohr 18 vorgesehene Öffnungen 44 erfolgt ein Duckausgleich zu dem Bereich oberhalb des Pegels 16 innerhalb des Behälters 12. Mit Absaugen der Flüssigkeit werden gleichzeitig die adulten Stammzellen, die über die Öffnung 20 in das Rohr 18 gelangen, mit abgesaugt. Das so gewonnene Flüssigkeits-/Stammzellen-Gemisch wird in einem weiteren hier angedeuteten Behälter 46 überführt, innerhalb dem dann die Stammzellen aus der Flüssigkeit herausgefiltert werden können.

Zur Erhöhung der Effektivität kann der Strömungskanal der Austrittsanordnung 30 unterschiedlich konturierte Strömungsflächen aufweisen. Diese können beispielsweise zur Ausbildung wenigstens einer Düse führen. Auch können Umlenkflächen vorhanden sein, die ein verbessertes Anströmen des Fettgewebes durch den Fluidstrahl 38 ermöglichen. Hierdurch wird ein verbessertes Abwaschen der adulten Stammzellen von den Fettzellen ermöglicht.

Figur 3 zeigt eine schematische Draufsicht auf die Austrittsanordnung 30. Es wird deutlich, dass die Austrittsanordnung 30 aus einem Hohlzylinder 50 besteht, in dessen Zentrum die Zuführeinrichtung 28 mündet. Eine Fixierung zwischen der Zuführeinrichtung 28 und dem Hohlzylinder 50 kann über hier angedeutete Stege 52 erfolgen. Die Darstellung in Figur 3 zeigt einen Einblick in die Austrittsanordnung 30 aus Richtung der Ausgangsöffnung 36. Die Eingangsöffnung 34 wird durch den verbleibenden Ringspalt zwischen der Austrittsanordnung 28 und dem Hohlzylinder 50 gebildet. Durch diese Eingangsöffnung 34 gelangt das Fettgewebe mit den Fettzellen und den adulten Stammzellen in die Austrittanordnung 30. Der Fluidstrahl 38 tritt mittig in die Austrittsanordnung 30 ein, so dass ein symmetrisches Mitreißen des Fettgewebes in die Austrittanordnung 30 erfolgt. Innerhalb der Austrittsanordnung 30 trifft der Fluidstrahl 38 dann auf das Fettgewebe und wäscht die an den Fettzellen anhaftenden adulten Stammzellen ab.

Figur 4A zeigt eine schematische Schnittdarstellung durch eine abgewandelte Ausführungsform der Austrittsanordnung 30. Es wird deutlich, dass der Hohlzylinder 50 in seinem Strömungsraum 54 eine Engstelle 56 ausbildet. Hierdurch kommt es zur Ausbildung von Strömungsflächen 58, die einerseits von dem Fluidstrahl 38 und dem mitgeführten Fettgewebe angeströmt werden. Es kommt an den Strömungsflächen 58 zu Verwirbelungen, so dass mittels des Fluidstrahles 38 ein besseres Auswaschen der an den Fettzellen anhaftenden adulten Stammzellen möglich ist.

Figur 4B ist eine weitere Ausführungsvariante einer Austrittsanordnung 30 gezeigt. Hier mündet in den Hohlzylinder 50 ein Bypass 60, der eine Öffnung zu dem Fettgewebe innerhalb des Behälters 12 aufweist. Bei Eintritt des Fluidstrahles 38 in den Innenraum des Hohlzylinders 50 bildet der Bypass 60 eine sogenannte Venturidüse aus, das heißt der Fluidstrahl 38 reißt über den Bypass 60 eine zusätzliche Volumenmenge an Fettgewebe mit. Hierdurch kann die Effektivität der Austrittsanordnung 30 beim Auswaschen der an den Fettzellen anhaftenden adulten Stammzellen erhöht werden.

Figur 5 zeigt stark schematisiert eine Ansammlung von Fettzellen 62 an deren Oberfläche adulte Stammzellen 64 anhaften. Durch Auftreffen des stilisiert dargestellten Fluidstrahls 38 erfolgt ein mechanisches Abwaschen der Stammzellen 64 von der Oberfläche der Fettzellen 62. Zwischen den Stammzellen 64 und den Fettzellen 62 besteht damit keine Haftung mehr, so dass die Stammzellen 64 aufgrund ihrer größeren Dichte innerhalb des Behälters 12 nach unten sinken (Figur 1).

### Bezugszeichenliste

- 10: Vorrichtung
- 12: Behälter
- 14: Anschlussstutzen
- 16: Pegel
- 18: Rohr
- 20: Mündung
- 22: Boden
- 24: Schicht von Fettzellen
- 26: Schicht der Flüssigkeit
- 28: Zuführeinrichtung
- 30: Austrittsanordnung
- 32: Pfeile
- 34: Eingangsöffnung
- 36: Ausgangsöffnung
- 38: Fluidstrahl
- 39: Pfeil
- 40: Saugstutzen
- 42: Saugmündung
- 44: Öffnungen
- 46: Behälter
- 50: Hohlzylinder
- 54: Strömungsraum
- 56: Engstelle
- 58: Strömungsflächen
- 60: Bypass
- 62: Fettzellen
- 64: Stammzellen

## Patentansprüche

1. Vorrichtung zum Separieren von adulten Stammzellen aus Fettgewebe,
**gekennzeichnet durch**
einen Behälter (12) zur Aufnahme von aus einer biologischen Struktur entnommenem Fettgewebe, das Fettzellen (62) und Stammzellen (64) umfasst,
eine Zuführeinrichtung (28) für ein unter Druck stehendes Fluid, eine Austrittsanordnung (30) für das unter Druck stehende Fluid, wobei die Austrittsanordnung von einem Hohlkörper gebildet wird und die Austrittsanordnung innerhalb des Behälters (12) derart angeordnet ist, dass der die Austrittsanordnung bildende Hohlkörper eine Ausgangsöffnung (36) und eine Eingangsöffnung (34) besitzt, in die das die Fettzellen und die Stammzellen enthaltende Fettgewebe **durch** den Fluidstrahl eingesaugt wird.

2. Vorrichtung nach Anspruch 1,
**dadurch gekennzeichnet, dass**
der Hohlkörper als Hohlzylinder (50) ausgebildet ist.

3. Vorrichtung nach einem der vorhergehenden Ansprüche 1 oder 2,
**dadurch gekennzeichnet, dass**
die Austrittsanordnung (30) Strömungsflächen (58) aufweist.

4. Vorrichtung nach einem der vorhergehenden Ansprüche 1 bis 3,
**dadurch gekennzeichnet, dass**
die Austrittsanordnung (30) eine Düse, insbesondere eine Venturidüse, ausbildet.

5. Verfahren zum Separieren von adulten Stammzellen aus Fettgewebe mit folgenden Verfahrensschritten:
Einbringen eines die Stammzellen und das Fettgewebe umfassenden Fettgewebeflüssigkeitsgemisches in eine Vorrichtung gemäß Anspruch 1,
Zuführen eines unter Druck zwischen 2 und 10 bar stehenden Fluidstrahles in das Fettzellen und Stammzellen enthaltende Fettgewebe,
Trennen der Stammzellen von den Fettzellen durch eine Strahlwirkung des Fluidstrahls,
Separieren der Stammzellen.

6. Verfahren nach Anspruch 5,
**dadurch gekennzeichnet, dass**
ein Stammzellen-Fluid-Gemisch abgesaugt wird.

7. Verfahren nach einem der vorhergehenden Ansprüche 5 oder 6,
**dadurch gekennzeichnet, dass**
die adulten Stammzellen aus dem Stammzellen-Fluid-Gemisch gefiltert werden.

## Claims

1. An apparatus for separating adult stem cells from adipose tissue,
**characterized by**
a container (12) for holding adipose tissue removed from a biological structure, which comprises fatty cells (62) and stem cells (64),
a feed installation (28) for a fluid under pressure, an outlet arrangement (30) for the fluid under pressure, wherein the outlet arrangement is formed by a hollow body and the outlet arrangement is disposed within the container (12) such that the hollow body that forms the outlet arrangement has an exit opening (36) and an entrance opening (34) into which the adipose tissue containing the fatty cells and the stem cells is sucked in by the fluid jet.

2. The apparatus according to claim 1,
**characterized in that**
the hollow body is formed as a hollow cylinder (50).

3. The apparatus according to any one of the preceding claims 1 or 2,
**characterized in that**
the outlet arrangement (30) has flow surfaces (58).

4. The apparatus according to any one of the preceding claims 1 to 3,
**characterized in that**
the outlet arrangement (30) forms a nozzle, in particular a venturi nozzle.

5. A method for separating adult stem cells from adipose tissue, comprising the following method steps:
introducing an adipose tissue fluid mixture comprising the stem cells and the fatty tissue into an apparatus according to claim 1,
adding a fluid jet under pressure of between 2 and 10 bar into the adipose tissue containing fatty cells and stem cells,
dividing the stem cells from the fatty cells through a jet impact of the fluid jet,
separating the stem cells.

6. The method according to claim 5,
**characterized in that**
a stem cell-fluid mixture is sucked away.

7. The method according to any one of the preceding claims 5 or 6,
**characterized in that**
the adult stem cells are filtered from the stem cell-fluid mixture.

## Revendications

1. Dispositif de séparation de cellules souches adultes du tissu adipeux,
**caractérisé par**
un réservoir (12) destiné à contenir du tissu adipeux prélevé dans une structure biologique et contenant des cellules adipeuses (62) et des cellules souches (64),
un dispositif d'amenée (28) pour un fluide sous pression, un dispositif de sortie (30) pour ledit fluide sous pression, le dispositif de sortie étant formé par un corps creux et étant disposé à l'intérieur du réservoir (12) de telle sorte que le corps creux formant le dispositif de sortie comporte
un orifice de sortie (36) et un orifice d'entrée (34), dans lesquels le tissu adipeux contenant les cellules adipeuses et les cellules souches est aspiré par le biais du jet de fluide.

2. Dispositif selon la revendication 1,
**caractérisé en ce que**
le corps creux est réalisé sous forme de cylindre creux (50).

3. Dispositif selon l'une des revendications 1 et 2,
**caractérisé en ce que**
le dispositif de sortie (30) présente des zones d'écoulement (58).

4. Dispositif selon l'une des revendications 1 à 3,
**caractérisé en ce que**
le dispositif de sortie (30) forme une buse, notamment une buse à effet Venturi.

5. Procédé de séparation de cellules souches adultes du tissu adipeux selon les étapes suivantes :
introduction d'un mélange fluide de tissu adipeux contenant les cellules souches et le tissu adipeux dans un dispositif
selon la revendication 1 ;
introduction d'un jet de fluide soumis à une pression comprise entre 2 et 10 bars dans le tissu adipeux contenant les cellules adipeuses et les cellules souches ;
séparation des cellules souches des cellules adipeuses par l'écoulement du jet de fluide ;
séparation des cellules souches.

6. Procédé selon la revendication 5,
**caractérisé en ce que**
un mélange fluide de cellules souches est aspiré.

7. Procédé selon l'une des revendications 5 ou 6,
**caractérisé en ce que**
les cellules souches adultes sont filtrées à partir du mélange fluide de cellules souches.
